Europäisches Patentamt

(19) **European Patent Office**

Office européen des brevets

(11) Publication number: **0 241 894**
**B1**

(12) EUROPEAN PATENT SPECIFICATION

(45) Date of publication of the patent specification:
**16.08.90**

(51) Int. Cl.⁵: $G01N\ 3/08$

(21) Application number: **87105440.9**

(22) Date of filing: **13.04.87**

(54) Yarn testing method and apparatus.

(30) Priority: **15.04.86 ZA 862796**

(43) Date of publication of application:
**21.10.87 Bulletin 87/43**

(45) Publication of the grant of the patent:
**16.08.90 Bulletin 90/33**

(84) Designated Contracting States:
**BE CH DE ES FR GB IT LI**

(56) References cited:
**AT-B- 347 150**
**US-A- 4 148 218**

**L'INDUSTRIE TEXTILE, no. 979, May 1969,**
**pages 323-327; "Le contrôle des fils par**
**l'extensiomètre automatique I.T.F-M."**

(73) Proprietor: **ZELLWEGER USTER AG, Wilstrasse 11,**
**CH-8610 Uster(CH)**

(72) Inventor: **CIZEK, Jaromir, 5 Harold Road Charlo, Port**
**Elizabeth, 6000 Cape Province(ZA)**

## Description

The present invention relates to a method for determining tensile properties of yarns by feeding a predetermined length of yarn discontinuously from a holder to a pair of spaced clamps, clamping the length of yarn by the said clamps, causing one or both clamps to move relative to the other progressively to tension the yarn, and measuring the yarn tension or the elongation of the length of yarn during the tensioning process.

The method according to the invention will in particular be suitable for determining the suitability of yarns for weaving and knitting purposes. It is however envisaged that the term "yarn" herein includes any alongated fibre, strand, string, sliver or the like. The method enables not only determining the tensile properties like the tension and/or the tensile strength of yarns but can also give a statistical indication of the number of weak points per length unit of a yarn.

In known tensile strength testing apparatus like the one described in U.S.A 4 338 824 the yarn to be tested is discontinuously drawn off from a holder, passed over guiding means and inserted into the actual testing track between a pair of spaced clamps. After the clamps have been closed they are moved one relative to the other whereby the yarn tension increases. The clamps exert a load to the yarn until the yarn breaks. After having removed the broken yarn from the apparatus the method is repeated for the next yarn length to be tested and so on.

Because of its intermittent course this known yarn testing method has a very small processing velocity. This is a disadvantage as tensile strength testing apparatus are used in textile laboratories together with other testing apparatus like eveness and count testers which have a much higher processing velocity.

The present invention seeks to avoid this disadvantage by providing a method for determining tensile properties of yarns which enables a higher processing velocity than known methods.

The method according to the invention is characterised in drawing off the yarn continuously from the holder, providing clamps which comprise each a rotable disc, clamping the yarn to the discs over an arc portion of the periphery thereof which is less than the length of the periphery so that the yarn is intermittently clamped and released, and guiding the yarn over a sensor for measuring the tension or the elongation of the yarn, said sensor being arranged between the clamps.

The invention further relates to an apparatus for carrying out the method according to the invention. This apparatus is characterised by a yarn storage device arranged between a yarn holder and clamps, by holding means for clamping the yarn to an arc portion of the periphery of discs, and by a yarn conduit arranged between the clamps and comprising a sensor for measuring the tension or the elongation of the yarn.

Apparatus dealing with yarn tensile and using rotatable discs for clamping the yarn are described in "L'INDUSTRIE TEXTILE", No. 797, May 1969, pages 323-327 and in US-A 4 148 218. But these apparatus do not describe any tensile testing installations but apparatus for applying tensile stress to yarn or fibre. The yarn is always fixedly clamped to the discs and there cannot be seen how it could be threaded into the "clamps " after having been broken during tensile testing. Therefore, the apparatus described in these references are not suitable for measuring the tensile force or elongation values.

In order to more clearly illustrate the invention an embodiment thereof is described hereunder purely by way of example with reference to the accompanying drawings wherein:

Fig. 1 is a schematic perspective view of an apparatus according to the invention;
Fig. 2 is an exploded view of a portion of the apparatus of Fig. 1;

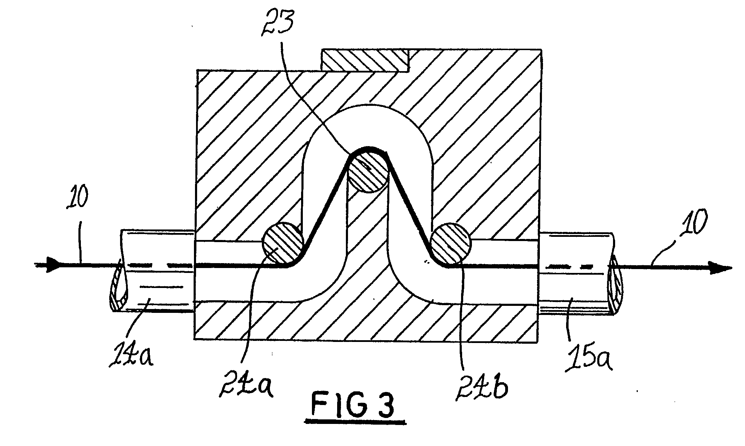

Fig. 3 is a sectioned elevation of a force transducer of the apparatus of Fig. 1;
Fig. 4 is an elevational view of the apparatus of Fig. 1;
Fig. 5 is a plan view of the apparatus of Fig. 1;
Fig. 6 is an enlarged view of a section on line VI-VI of Fig. 4; and
Fig. 7 is an enlarged partially sectioned elevational view of a portion of the apparatus of Fig. 4.

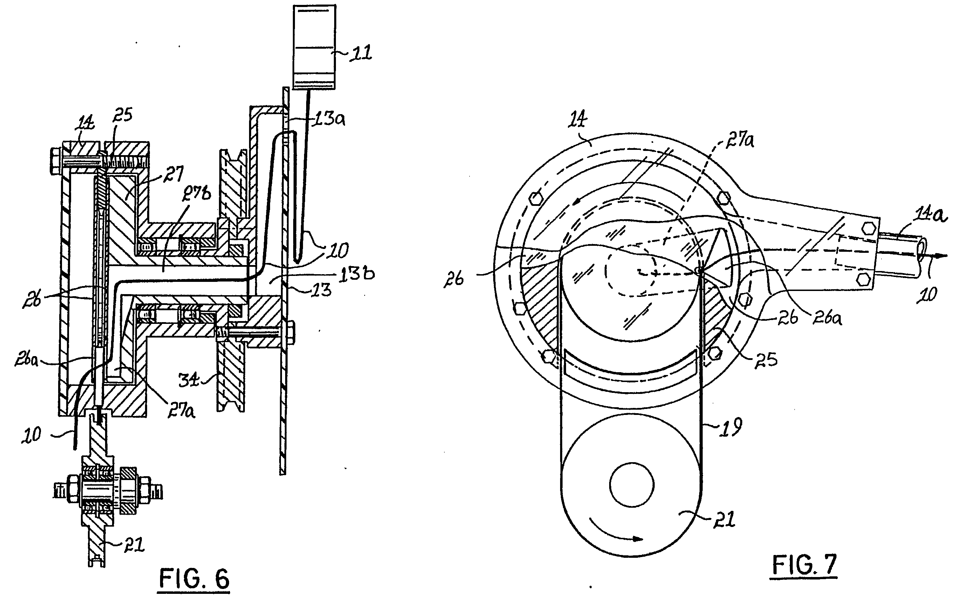

Referring to the drawings the apparatus in accordance with the invention for testing yarn 10 comprises a yarn pathway which starts with a spool 12 or other suitable reservoir for yarn, and which terminates in an outlet 16 which is coupled to a suction device, not shown. The yarn 10 is continually fed from the spool 12 by means of a pair of rollers 11 and during a feed cycle, a predeterminded length of yarn which has passed through the rollers 11, is sucked along the yarn pathway to be tested during a testing cycle which is described in more detail below. In order to prevent knotting of the yarn during the feed cycle, an eccentric device is provided to guide the yarn from a position remote from the feed rollers 11 towards a position which proximates the rollers 11 and thus serves to take up slack during the feed cycle. With reference to Figure 6 it will be noted that the eccentric comprises a plate 13 which has an eccentric aperture 13a therein which defines an inlet to the suction pathway 13b for the yarn.

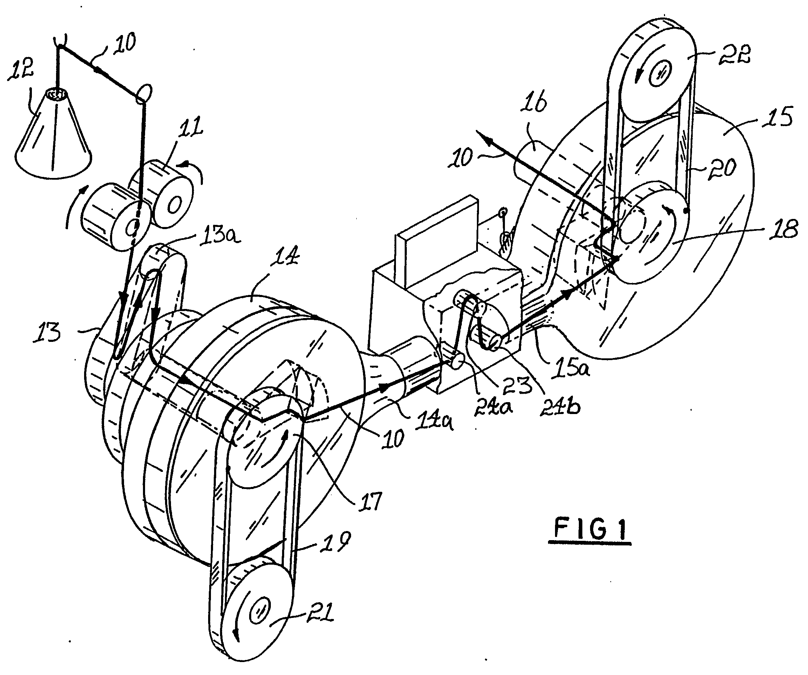

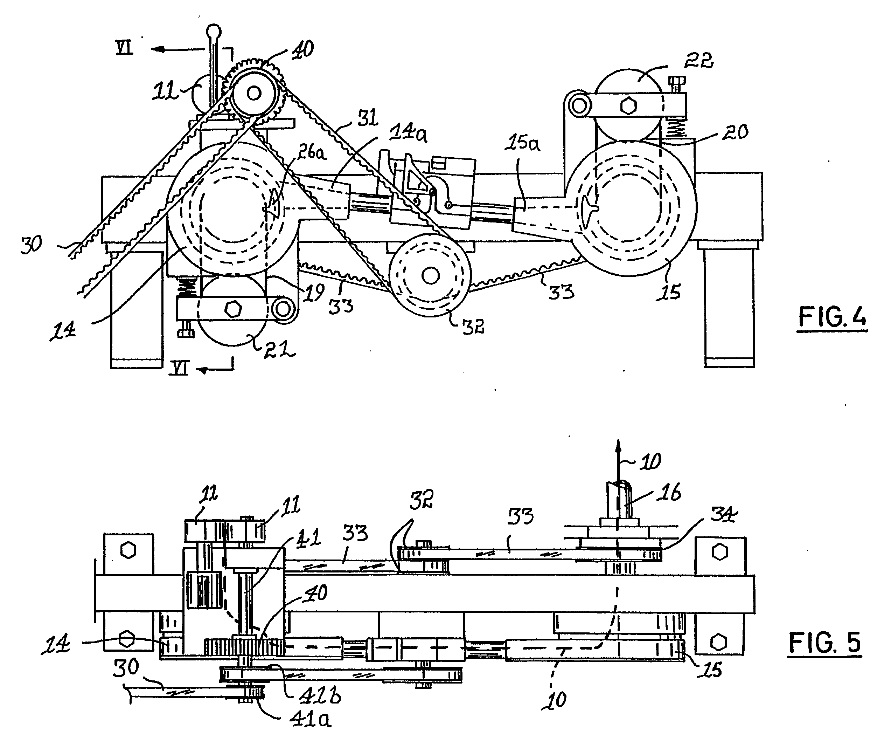

The yarn pathway 13b leads to a pair of spaced clamps in the form of pulleys 17 and 18 over which belts 19 and 20 respectively run. The belts 19 and 20 are of the endless variety and extend between the pulleys 17 and 18 and spaced idlers 21 and 22 respectively. During the testing cycles mentioned above, yarn is fed into the nip defined by the belts 19, 20 and the pulleys 17, 18, and thus clamped. During further rotation of the pulleys 17 and 18 in the direction shown in Figure 1, the yarn is tensioned until breakage occurs. During the tensioning stage the tension in the yarn 10 is measured by a force transducer 23 which is located in the yarn pathway. In the zone of the force transducer 23, the yarn pathway follows a half-wave curve with the yarn passing around a roller 24a over the transducer 23 and back around a roller 24b. During the tensioning process, the elongation in the yarn 10 will also be measured by means of a slotted pulse generator 40, Figure 4, 5, which is coupled in the drive train for the apparatus. By counting the number of pulses given by the generator 40 during the period when the force measured by the transducer 23 is greater than zero, the elongation of the yarn can be determined.

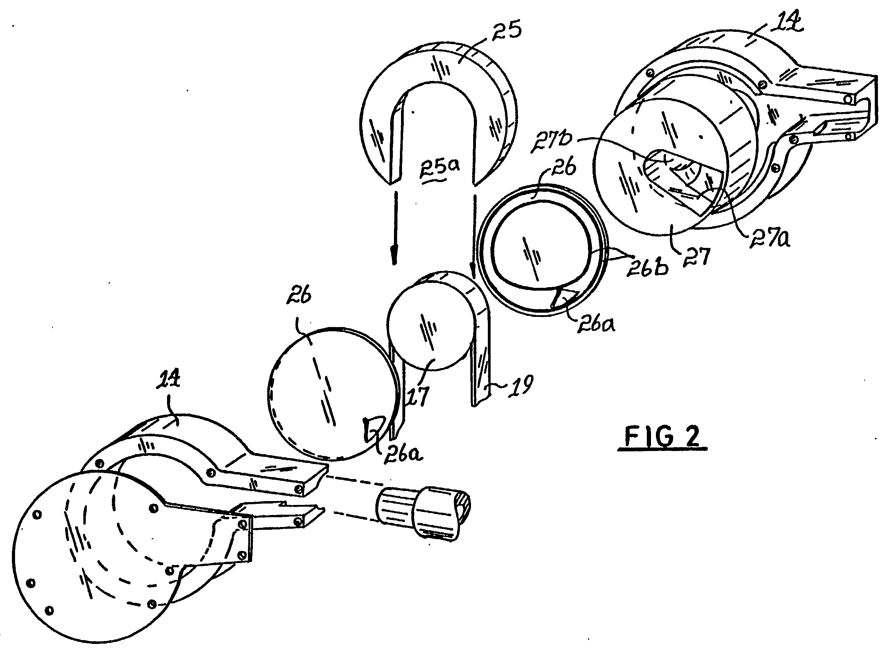

The clamping and tensioning pulleys 17 and 18 are located in spaced housings 14 and 15, which define an outlet 14a and an inlet 15a respectively, the outlet 14a and inlet 15a being coupled in the pathway leading to and from force transducer zone, Fig. 1. Also rotatable within the housing 14 in conjunction with the pulley 17, is a disc 27 which defines an axial portion 27b which extends from the portion 13b of the yarn pathway described above. From the axial portion 27b the yarn pathway extends radially along a cut-out 27a in the disc 27 to beyond the periphery of a pulley 17. At the outer extremity of the cut-out 27a, the latter aligns with apertures 26a in a pair of plate elements 26 which flank the pulley 17 and rotate in conjunction therewith. A horse-shoe shaped disc 25 fits between the plates 26 and defines a cut-out 25a therein so that the plate 25 seals off the apertures 26a in all angular positions of the plates 26 except when the apertures 26a align with the cut-out 25a. Thus, during the period when the apertures 26a align with the cut-out 25a, the yarn pathway will be opened and yarn will be sucked therealong by means of the suction device. With reference to Figure 2 it will be noted that the cut-out 25a corresponds with a 180° portion of the pulley 17 which is not in engagement with the belt 19. The feeding and suction cycle thus commences when the pulley 17 and belt 19 release a length of yarn, and the cycle ceases, when the belt and pulley again clamp a length of yarn at the onset of a tensioning cycle. With this arrangement, suction at the outlet 16 will be continuous, with suitable seals such as that shown at 26b on the plates 26 being provided effectively to shut-off airflow during the tensioning cycle.

As mentioned above the disc 27, pulley 17, plates 26, and eccentric plate 13, will also rotate in conjunction and for this purpose a common drive pulley 34, Figure 6, is provided. The drive train of the apparatus is shown more clearly in Figure 4 and Figure 5, and comprises a belt 30 driven by a suitable drive motor, not shown. The belt 30 drives a shaft 41 via a pulley 41a, with the shaft 41 driving the pulse generator 40, feedrollers 11, and a pulley 41b. The latter pulley in turn drives via a belt 31 a pulley 32 which drives a belt 33 which in turn drives pulleys 34 for appropriate rotation of the clamping pulleys 17 and 18.

The advantages of the arrangement of the invention will be apparant to persons skilled in the art. The apparatus provides a continuous yarn feed with extremely rapid threading and tensioning of the yarn to breakage. With the arrangement of the invention yarn can be tested at a rapid rate to provide an indication of the level and frequency of weak place in the yarn and therefore the expected weaving performance of the yarn.

## Claims

1. Method for determining tensile properties of yarns by feeding a predetermined length of yarn (10) discontinuously from a holder (12) to a pair of spaced clamps (17, 19; 18, 20), clamping the length of yarn by the said clamps, causing one or both clamps to move relative to the other progressively to tension the yarn, and measuring the yarn tension or the elongation of the length of yarn during the tensioning process, characterised in drawing off the yarn (10) continuously from the holder (12), providing clamps (17, 19; 18, 20) which comprise each a rotatable disc (17, 18), clamping the yarn to the discs over an arc portion of the periphery thereof which is less than the length of the periphery so that the yarn is intermittently clamped and released, and guiding the yarn over a sensor (23) for measuring the tension or the elongation of the yarn, said sensor being arranged between the clamps.

2. Method according to claim 1, characterised in that the sensor (23) is displaced out of a linear path between the clamps (17, 19; 18, 20).

3. Method according to claim 2, characterised in that the yarn (10) is caused to follow a generally half-wave curve pathway and the sensor (23) is disposed at the wave crest and is displaceable under tension of the yarn towards the wave base.

4. Method according to claim 1, characterised in that the tensioning of the yarn (10) is effected by rotating the discs (17, 18) once the yarn has been clamped, with the rotational axis of the discs being disposed transverse to the direction of the yarn.

5. Method according to any one of claims 1 to 4, wherein the yarn (10) is fed by means of a fluid stream.

6. Apparatus for carrying out the method according to claim 1, characterised by a yarn storage device (13, 13a) arranged between a yarn holder (12) and clamps (17, 19: 18, 20), by holding means (19, 20) for clamping the yarn (10) to an arc portion of the periphery of discs (17, 18), and by a yarn conduit (14a, 15a) arranged between the clamps and comprising a sensor for measuring the tension or the elongation of the yarn (23) .

7. Apparatus according to claim 6, characterised in that the holding means (19, 20) are in the form of belts (19, 20) each being draped over the said portion of the periphery of the discs (17, 18) whereby the yarn (10) is clamped between each disc and its belt by feeding the yarn into the rip between belt and disc and by rotating the disc.

8. Apparatus according to claim 6, characterised in that the sensor (23) is a force transducer.

9. Apparatus according to claim 7 or 8, characterised in a pair of spaced guide formations (24a, 24b) arranged in the linear path between the clamps (17, 19; 18, 20) with the sensor (23) provided in the zone of the guide formations and that the yarn (10) is guided to follow a U-shaped path between a first guide formation (24a), the sensor (23), and a second guide formation (24b).

10. Apparatus according to any one of claims 6 to 9, characterised in a fluid stream for feeding the yarn through the conduit (14a, 15a).

11. Apparatus according to claim 10, characterised in a pair of feed rollers (11) rotating at a predetermined speed, with the conduit being adapted to be opened periodically to supply yarn from the feed rollers (11) into the pathway.

12. Apparatus according to claim 11, characterised in that the yarn storage device is formed by an eccentric device (13) provided to take up slack of the yarn (10) drawn off from the holder (12) while the yarn conduit is closed.

## Patentansprüche

1. Verfahren zur Bestimmung von Dehnungseigenschaften von Fäden durch diskontinuierliche Zuführung einer vorbestimmten Fadenlänge (10) von einem Vorrat (12) zu einem Paar beabstandeter Klemmen (17, 19; 18, 20), durch Festklemmung der Fadenlänge durch die genannten Klemmen, durch Einwirkung auf eine der oder beide Klemmen, sich relativ zueinander fortschreitend zu bewegen, um den Faden zu spannen, und durch Messung der Fadenspannung oder der Dehnung der Fadenlänge während des Spannungsvorgangs, dadurch gekennzeichnet, dass der Faden (10) vom Vorrat (12) kontinuierlich abgezogen wird, dass Klemmen (17, 19; 18, 20) vorgesehen werden, welche jede eine drehbare Scheibe (17, 18) umfassen, dass der Faden mit den Scheiben über einen Bogenteil von deren Umfang verklemmt wird, welcher kleiner ist als die Länge des Umfangs, so dass der Faden intermittierend festgeklemmt und freigegeben wird, und dass der Faden über einen Sensor (23) zur Messung der Spannung oder der Dehnung des Fadens geführt wird, welcher Sensor zwischen den Klemmen angeordnet ist.

2. Verfahren nach Anspruch 1, dadurch gekennzeichnet, dass der Sensor (23) ausserhalb einer geradlinigen Bahn zwischen den Klemmen (17, 19; 18, 20) angeordnet wird.

3. Verfahren nach Anspruch 2, dadurch gekennzeichnet, dass der Faden (10) veranlasst wird, einer im allgemeinen kurvenförmigen Bahn von der Form einer Halbwelle zu folgen, und dass der Sensor (23) am Wellengipfel angeordnet und unter der Spannung des Fadens gegen den Wellengrund verstellbar ist.

4. Verfahren nach Anspruch 1, dadurch gekennzeichnet, dass das Spannen des Fadens (10) durch Rotation der Scheiben (17, 18) nach dem Festklemmen des Fadens erfolgt, wobei die Rotationsachse der Scheiben quer zur Richtung des Fadens angeordnet ist.

5. Verfahren nach einem der Ansprüche 1 bis 4, bei welchem der Faden (10) mittels einer Fluid-Strömung gefördert wird.

6. Vorrichtung zur Durchführung des Verfahrens nach Anspruch 1, gekennzeichnet durch einen zwischen einem Fadenvorrat (12) und Klemmen (17, 19; 18, 20) angeordneten Fadenspeicher (13, 13a), durch Haltemittel (19, 20) zum Verklemmen des Fadens (10) mit einem Bogenteil des Umfangs von Scheiben (17, 18), und durch einen zwischen den Klemmen angeordneten Fadenkanal (14a, 15a), welcher einen Sensor (23) zur Messung der Spannung oder der Dehnung des Fadens enthält.

7. Vorrichtung nach Anspruch 6, dadurch gekennzeichnet, dass die Haltemittel (19, 20) durch Riemen (19, 20) gebildet sind, von denen jeder über den genannten Teil des Umfangs der Scheiben (17, 18) gelegt ist, wobei der Faden (10) zwischen jeder Scheibe (17, 18) und ihrem Riemen durch Zuführen des Fadens in den Spalt zwischen Riemen und Scheibe und durch Drehung der Scheibe festgeklemmt wird.

8. Vorrichtung nach Anspruch 6, dadurch gekennzeichnet, dass der Sensor (23) ein Kraftwandler ist.

9. Vorrichtung nach Anspruch 7 oder 8, gekennzeichnet durch ein Paar beabstandeter, in der geradlinigen Bahn zwischen den Klemmen (17, 19; 18, 20) angeordneter Führungsmittel (24a, 24b), in deren Bereich der Sensor (23) vorgesehen und der Faden (10) so geführt ist, dass er einer U-förmigen Bahn zwischen einem ersten Führungsmittel (24a), dem Sensor (23) und einem zweiten Führungsmittel (24b) folgt.

10. Vorrichtung nach einem der Ansprüche 6 bis 8, gekennzeichnet durch eine Fluid-Strömung zur Förderung des Fadens durch den Kanal (14a, 15a).

11. Vorrichtung nach Anspruch 10, gekennzeichnet durch ein Paar von Förderwalzen (11), welche mit einer vorbestimmten Geschwindigkeit rotieren, wobei der Kanal so eingerichtet ist, dass er periodisch geöffnet wird, um Faden von den Förderwalzen (11) in die Bahn zu liefern.

12. Vorrichtung nach Anspruch 11, dadurch gekennzeichnet, dass der Fadenspeicher durch eine exzentrische Einrichtung (13) gebildet ist, welche zur Aufnahme des bei geschlossenem Kanal vom Vorrat (12) abgezogenen losen Fadens (10) vorbereitet ist.

## Revendications

1. Procédé pour déterminer les propriétés de traction de fils en introduisant une longueur prédéterminée de fil (10) de manière discontinue d'un support (12) vers une paire de serre-fils espacés (17, 19; 18, 20), en serrant la longueur du fil par lesdits serre-fils, en forçant l'un ou les deux serre-fils à se déplacer relativement à l'autre, progressivement, pour mettre le fil sous tension et en mesurant la tension du fil ou l'allongement de la longueur du fil pendant le processus de mise sous tension, caractérisé en ce que l'on tire continuellement le fil (10) du support (12), on prévoit des serre-fils (17, 19; 18, 20) dont chacun comprend un disque rotatif (17, 18), on serre le fil sur les disques sur une portion d'arc de leur pourtour qui est plus petite que la longueur du pourtour, donc le fil est par intermittence serré et libéré et on guide le fil sur un capteur (23) pour mesurer la tension du l'allongement du fil, ledit capteur étant agencé entre les serre-fils.

2. Procédé selon la revendication 1, caractérisé en ce que le capteur (23) est décalé par rapport à un trajet linéaire entre les serre-fils (17, 19; 18, 20).

3. Procédé selon la revendication 2, caractérisé en ce que le fil (10) est forcé à suivre une courbe généralement à une seule alternance et le capteur (23) est disposé à la crête de l'onde et peut être déplacé, sous la tension du fil, vers la base de l'onde.

4. Procédé selon la revendication 1, caractérisé en ce que la mise sous tension du fil (10) est effectuée par rotation des disques (17, 18) lorsque le fil a été serré, l'axe de rotation des disques étant disposé transversalement à la direction du fil.

5. Procédé selon l'une quelconque des revendications 1 à 4, où le fil (10) est fourni par un courant de fluide.

6. Dispositif pour la mise en œuvre du procédé selon la revendication 1, caractérisé par un mécanisme de stockage du fil (13, 13a) agencé entre un support du fil (12) et des serre-fils (17, 19; 18, 20), par des moyens de maintien (19, 20) pour serrer le fil (10) sur une portion d'arc du pourtour des disques (17, 18) et par un conduit du fil (14a, 15a) agencé entre les serre-fils et comprenant un capteur (23) pour mesurer la tension ou l'allongement du fil.

7. Dispositif selon la revendication 6, caractérisé en ce que les moyens de maintien (19, 20) ont la forme de courroies (19, 20), chacune passant sur ladite portion du pourtour des disques (17, 18) pour qu'ainsi le fil (10) soit serré entre chaque disque et sa courroie en introduisant le fil dans l'ouverture entre la courroie et le disque et en faisant tourner le disque.

8. Dispositif selon la revendication 6, caractérisé en ce que le capteur (23) est un transducteur de force.

9. Dispositif selon la revendication 7 ou 8, caractérisé par une paire de formations espacées de guidage (24a, 24b) agencées dans le trajet linéaire entre les serre-fils (17, 19; 18, 20) avec le capteur (23) prévu dans la zone des formations de guidage et en ce que le fil (10) est guidé pour suivre un trajet en forme de U entre une première formation de guidage (24a), le capteur (23) et une seconde formation de guidage (24b).

10. Dispositif selon l'une quelconque des revendications 6 à 9, caractérisé par un courant de fluide pour introduire le fil à travers le conduit (14a, 15a).

11. Dispositif selon la revendication 10, caractérisé par une paire de rouleaux d'alimentation (11) tournant à une vitesse prédéterminée, le conduit étant adapté à être ouvert périodiquement pour fournir le fil des rouleaux d'alimentation (11) dans le trajet.

12. Dispositif selon la revendication 11, caractérisé en ce que le mécanisme de stockage du fil est formé par un mécanisme excentrique (13) prévu pour reprendre le jeu du fil (10) tiré du support (12) tandis que le conduit du fil est fermé.

FIG 1

25

14

25a

27b

26

27a

26b 27

26a

26

14

17

19

26a

**FIG 2**

EP 0 241 894 B1

FIG 3

EP 0 241 894 B1

FIG. 4

FIG. 5

EP 0 241 894 B1

FIG. 6

FIG. 7

EP 0 241 894 B1